# EUROPÄISCHE PATENTSCHRIFT

(11) **EP 4 543 367 B1**
(45) Veröffentlichungstag und Bekanntmachung des Hinweises auf die Patenterteilung: **06.05.2026**
(21) Anmeldenummer: 23732584.0
(22) Anmeldetag: 14.06.2023
(51) Int. Cl.: A61F 2/04, A61F 2/06

(54) **IMPLANTAT UND ANORDNUNG AUFWEISEND EINE STRAHLUNGSQUELLE UND EIN IMPLANTAT**
IMPLANT AND ASSEMBLY HAVING A RADIATION SOURCE AND AN IMPLANT
IMPLANT ET ENSEMBLE COMPORTANT UNE SOURCE DE RAYONNEMENT ET UN IMPLANT

(30) Priorität: 21.06.2022 DE 102022115394
(43) Veröffentlichungstag der Anmeldung: 30.04.2025
(73) Patentinhaber: Rheinisch-Westfälische Technische Hochschule (RWTH) Aachen, 52062 Aachen (DE)
(72) Erfinder: BAUER, Benedict, 52070 Aachen (DE); GRIES, Thomas Gerhard, 52072 Aachen (DE); SLABU, Ioana, 52070 Aachen (DE); SCHMITZ-RODE, Thomas, 52070 Aachen (DE)
(74) Vertreter: Michalski Hüttermann & Partner Patentanwälte mbB
(86) Internationale Anmeldenummer: PCT/EP2023/065995
(87) Internationale Veröffentlichungsnummer: WO 2023/247294

(56) Entgegenhaltungen:
- EP-A1- 0 543 498
- EP-A1- 2 740 445
- CN-A- 102 371 006
- US-A1- 2008 071 353
- US-A1- 2018 117 154

## Beschreibung

Die vorliegende Erfindung betrifft ein Implantat, wie insbesondere einen Stent. Die vorliegende Erfindung betrifft insbesondere ein Implantat, welches bevorzugt in ein Hohlorgan oder ein Gefäß eines Körpers einführbar ist und welches besonders bevorzugte Eigenschaften aufweist. Die vorliegende Erfindung betrifft ferner eine Anordnung aufweisend ein derartiges Implantat und eine Strahlungsquelle zum Emittieren von elektromagnetischer Strahlung.

Biodegradierbare Polymerstents sind seit Langem Gegenstand der Forschung, da sie gegenüber konventionellen metallischen Stents, etwa aus Nitinol, nur temporär im Körper verbleiben. Denn in den meisten Anwendungen wird der Stent nur für kurze Zeit benötigt. Der dauerhafte Verbleib ist mit Nachteilen verbunden, wie der Gefahr der In-Stent-Restenose, neointimaler Hyperplasie und unvollständiger Heilung. Metallische Stents sind sehr schwer wieder zu entfernen und stellen im Falle der Restenose ein Problem dar. Biodegradierbare Polymerstents bieten großes Potenzial, sind jedoch bislang in ihrer Anwendung durch materialbedingte, geringe Radialkraft limitiert.

EP 3 277 375 B1 beschreibt, dass Eisen/Platin (Fe/Pt)-Partikel in einem Polymer dispergiert und in oder auf medizinische Geräte aufgebracht oder direkt mit diesen verbunden und magnetisiert werden können. Die magnetisierten Vorrichtungen werden verwendet, um magnetisch markierte Zellen anzuziehen, einzufangen und/oder auf der Oberfläche der Vorrichtung in vivo zu halten. Die magnetischen Vorrichtungen sind besonders nützlich, um Zellen, die den Belastungen und Kräften des biologischen Flüssigkeitsstroms ausgesetzt sind, einzufangen und festzuhalten, z. B. auf Stents, die nach einer Angioplastie implantiert werden, um eine patentierte Endotheloberfläche zu bilden, die das Auftreten einer Restenose verhindert. Sie können auch verwendet werden, um die Gewebsintegration an der Implantationsstelle einer Prothese, z. B. einer Hüft- oder Knieprothese mit Metalloberfläche, zu verbessern oder die Knochenerosion um eine Knochenschraube, einen Stift oder eine Platte aus Metall zu verringern. Die Vorrichtungen können aus einem Metall, einem Polymer oder einer Kombination davon hergestellt sein.

EP 2 249 804 B1 beschreibt Nanopartikel-enthaltende implantierbare und biologisch abbaubare Medizinprodukte und deren Verwendung zur thermotherapeutischen Nachbehandlung nach chirurgischer Entfernung von Tumoren und Krebsgeschwüren. Ein derartiges Medizinprodukt liegt insbesondere als Gewebe, Schwamm oder Film vor, um flexibel oder verformbar zu sein, wobei magnetische Partikel in dem Medizinprodukt enthalten sind, welche angeregt durch ein magnetisches Wechselfeld Wärme erzeugen und dadurch das Medizinprodukt erwärmen können.

US 2008/0071353 A1 beschreibt Stents, welche magnetisch induzierbare Nanopartikel enthalten, wobei die Partikel eine metallische Beschichtung aufweisen.

Ferner sei in diesem Zusammenhang auf das Dokument CN102371006 B verwiesen.

Derartige aus dem Stand der Technik bekannte Lösungen können noch Verbesserungspotential aufweisen, insbesondere hinsichtlich einer verbesserten Anwendbarkeit.

Es ist daher die Aufgabe der vorliegenden Erfindung, eine Maßnahme zu schaffen, durch welche wenigstens ein Nachteil des Stands der Technik zumindest teilweise überwunden wird. Es ist insbesondere eine Aufgabe der vorliegenden Erfindung, eine Maßnahme zu schaffen, mittels der die Anwendbarkeit eines Implantats, insbesondere eines Stents, verbessert werden kann.

Die Lösung der Aufgabe erfolgt erfindungsgemäß durch ein Implantat mit den Merkmalen des Anspruchs 1. Die Lösung der Aufgabe erfolgt erfindungsgemäß ferner durch eine Anordnung mit den Merkmalen des Anspruchs 13. Bevorzugte Ausgestaltungen der Erfindung sind in den Unteransprüchen, in der Beschreibung, und in den Figuren offenbart, wobei weitere in den Unteransprüchen oder in der Beschreibung oder den Figuren beschriebene oder gezeigte Merkmale einzeln oder in einer beliebigen Kombination einen Gegenstand der Erfindung darstellen können, wenn sich aus dem Kontext nicht eindeutig das Gegenteil ergibt.

Die vorliegende Erfindung betrifft ein Implantat zum Implantieren in einen Körper, insbesondere in ein Hohlorgan oder ein Gefäß eines Körpers, wobei das Implantat aus einem Filament aufgebaut ist, das wenigstens ein polymeres Matrixmaterial aufweist, in dem ein magnetisch heizbarer Füllstoff angeordnet ist, wobei das Filament einen Querschnitt mit einer Kern-Mantel-Struktur aufweist, wobei der Kern ein erstes, insbesondere polymeres, Matrixmaterial mit einem ersten Schmelzpunkt aufweist und wobei der Mantel ein zweites, polymeres Matrixmaterial mit einem zweiten Schmelzpunkt aufweist, wobei der magnetisch heizbare Füllstoff zumindest in dem zweiten Matrixmaterial vorliegt, wobei der zweite Schmelzpunkt niedriger ist, als der erste Schmelzpunkt, wobei der zweite Schmelzpunkt in einem Bereich liegt von ≥ 45 °C insbesondere bis ≤ 100 °C.

Ein derartiges Implantat weist gegenüber Lösungen aus dem Stand der Technik deutliche Vorteile auf, insbesondere hinsichtlich einer Kombination von einer guten Einbringbarkeit in ein Hohlorgan oder ein Gefäß eines Körpers im Zusammenspiel mit guten mechanischen Eigenschaften, insbesondere einer hohen Radialkraft nach dem Einbringen in das Hohlorgan oder das Gefäß.

Das hier beschriebene Implantat dient insbesondere dem Implantieren in einen Körper insbesondere eines Lebewesens, etwa in einen menschlichen Körper, wobei ein Implantieren in ein Gefäß oder Hohlorgan des Körpers, wie beispielhaft in eine Ader, die Luftröhre, Gallengänge sowie Harnleiter und -röhre, besonders bevorzugt ist. Hierzu kann das Implantat insbesondere eine gewisse Flexibilität aufweisen, um etwa in einem komprimierten und/oder verformten Zustand in den Körper, etwa in das Gefäß oder Hohlorgan, eingebracht zu werden und um an der gewünschten Position seine gewünschte Anwendungsform beziehungsweise Anwendungsgeometrie einzunehmen.

Darüber ist das Implantat insbesondere ein solches, welches nur temporär im Körper verbleibt und dabei zumindest teilweise, besonders bevorzugt vollständig aus biologisch abbaubaren Stoffen aufgebaut ist, also das erste und das zweite Matrixmaterial bevorzugt biodegradierbar sind. Unter einem bioabbaubaren (oder biodegradierbaren) Kunststoff ist dabei ein solcher zu verstehen, der dem grundsätzlichen Verständnis unterliegt, beispielsweise gemäß ASTM F2902-16.

Das Implantat ist aus einem Filament geformt, das auch als Faser bezeichnet werden kann. Zur Herstellung des Implantats kann das Filament in an sich bekannter Weise mit dem Fachmann geläufigen Faserverarbeitungsverfahren verarbeitet werden. Beispiele für derartige Faserverarbeitungsverfahren beziehungsweise textiltechnische Weiterarbeitungsprozesse umfassen etwa das Verkreuzen bzw. Verschlingen des Filaments, wie es beim Weben, Wirken, Stricken, der Spitzenherstellung, dem Flechten und Herstellung von getufteten Erzeugnissen geschieht. Ferner kann das Implantat ein Vlies sein, wobei es jedoch bevorzugt sein kann, dass das Filament eben kein Vlies ist.

Zuvor kann das Filament durch einen Spinnprozess erzeugt werden. Hierzu kann eine Coextrusion verwendet werden, um die Kern-Mantel-Struktur zu erzeugen. Grundsätzlich kann als Kern-Mantel-Struktur insbesondere eine Zweischichtstruktur vorliegen.

Insbesondere durch ein Coextrusions-Verfahren aber nicht beschränkt hierauf wird es möglich, auf Faserebene eine Kern-Mantel-Struktur zu erzeugen. Es ist bevorzugt, dass der Kern fadenförmig ausgestaltet ist und der Mantel eine schlauchförmige Struktur aufweist und den Kern zumindest teilweise umhüllt. In anderen Worten ist das Filament, aus welchem das Implantat insbesondere durch ein Faserverarbeitungsverfahren geformt wird, auf Faserebene als Mehrschichtstruktur ausgebildet. Der Kern ist fadenförmig und somit aus Vollmaterial geformt. Die Hülle ist schlauchförmig und um den Kern herum ausgebildet. Dies ist beispielsweise möglich, indem zwei koaxiale Düsen bei einem Extrusionsprozess verwendet werden, die im Inneren den Kern ausbilden und radial um den Kern herum den Mantel als schlauchförmige Struktur.

Unter einer schlauchförmigen Struktur soll dabei im Sinne der vorliegenden Erfindung insbesondere verstanden werden, dass der Mantel eine tubuläre Struktur aufweist, so dass der Mantel den Kern nach außen, also radial, insbesondere vollständig beziehungsweise vollflächig abdeckt, indem der Mantel um den Kern herum verläuft. Das Innere der Schlauchform ist dann insbesondere vollständig durch den Kern ausgefüllt. Insbesondere kann der Kern als auch der Mantel bevorzugt eine geschlossene Schicht ausbilden, wobei die Schichten bevorzugt frei von Poren sind. Poren im Kern oder im Mantel sollen aber grundsätzlich von der vorliegenden Erfindung umfasst sein.

Durch ein Coextrusionsverfahren zum Herstellen des Filaments kann eine besonders hohe Festigkeit erreicht werden, da die Materialien, insbesondere Polymermaterialien von Hülle und Kern, orientiert sind. Dies ist beispielsweise ein Vorteil zu einem additiven Herstellungsverfahren, bei dem die Materialien meist unorientiert sind.

Das Filament weist wenigstens ein polymeres Matrixmaterial auf, in dem ein magnetisch heizbarer Füllstoff angeordnet ist. Insbesondere kann der magnetisch heizbare Füllstoff in dem Matrixmaterial homogen fein verteilt vorliegen, wobei es bei dem hier beschriebenen Implantat vorgesehen sein kann, dass der Füllstoff nur in einem vordefinierten Bereich entlang des Querschnitts des Filaments vorliegt, wie dies nachstehend in größerem Detail beschrieben ist. Je homogener die Verteilung des Füllstoffs ist, desto homogener und definierter kann schließlich ein Aufheizen wie nachfolgend beschrieben ermöglicht werden.

Hinsichtlich des magnetisch heizbaren Füllstoffs soll dieser im Sinne der vorliegenden Erfindung insbesondere ein solcher sein, der sich ausgelöst durch ein Magnetfeld beziehungsweise ein elektromagnetisches Feld erhitzt. Das Erhitzen erfolgt dabei insbesondere definiert und reproduzierbar, so dass bei dem Anlegen eines Magnetfeldes mit bekannten Parametern eine derartige Wirkung erzielt wird, dass der Füllstoff beziehungsweise insbesondere das Filament auf einen definierten Temperaturwert aufheizbar ist. Es kann von besonderem Vorteil sein, wenn der Füllstoff derart aufheizbar ist, dass das Filament eine Temperatur aufweist in einem Bereich von 40°C bis 100°C, bevorzugt in einem Bereich von 45°C bis 100°C, wie nachstehend beschrieben.

Genauer umfasst der Kern ein erstes insbesondere polymeres Matrixmaterial mit einem ersten Schmelzpunkt und der Mantel umfasst ein zweites polymeres Matrixmaterial mit einem zweiten Schmelzpunkt. Entsprechend ist es bei dem hier beschriebenen Implantat vorgesehen, dass Kern und Mantel aus unterschiedlichen Materialien ausgebildet sind, welche sich in ihrem Schmelzpunkt unterscheiden.

Der Schmelzpunkt wird dabei insbesondere bestimmt bei 1,013 bar.

Hinsichtlich der Schmelzpunkte ist es genauer vorgesehen, dass der zweite Schmelzpunkt, also der Schmelzpunkt des Matrixmaterials der Hülle, niedriger ist, als der erste Schmelzpunkt, also der Schmelzpunkt des Matrixmaterials des Kerns, wobei der zweite Schmelzpunkt in einem Bereich liegt von ≥ 45 °C insbesondere bis ≤ 100 °C. Dabei ist bevorzugt eine Schmelzpunktdifferenz zwischen dem ersten Schmelzpunkt und dem zweiten Schmelzpunkt vorgesehen, die in einem Bereich liegt von ≥ 5 °C, beispielsweise ≥ 20 °C.

Hinsichtlich der Anordnung des magnetisch heizbaren Füllstoffs ist es ferner vorgesehen, dass dieser zumindest in dem zweiten Matrixmaterial vorliegt, also in anderen Worten in der Hülle. Dies ermöglicht insbesondere eine definierte Heizbarkeit der Hülle beziehungsweise des zweiten Matrixmaterials, so dass die Erhitzung lokal direkt in der Komponente stattfindet, die aufgeschmolzen oder zumindest angeschmolzen werden soll, wie nachstehend beschrieben. Bevorzugt kann der Füllstoff vorwiegend in dem äußeren Bereich vorliegen, wohingegen der Kern weniger des magnetisch heizbaren Füllstoffs aufweist beziehungsweise insbesondere frei von diesem ist. Somit ist es grundsätzlich bevorzugt, dass die Beladung des Füllstoffs in dem Mantel größer ist, als im Kern. Dadurch wird im Kern eine polymere Verstärkungsstruktur ausgebildet. Unter einer Verstärkungsstruktur des Kerns soll dabei im Sinne der vorliegenden Erfindung insbesondere verstanden werden, dass der Kern eine Verstärkung für den Mantel darstellt, insbesondere indem der Kern eine größere Stabilität beziehungsweise Festigkeit aufweist, als der Mantel. Insbesondere kann sich die Verstärkung auf die Zugfestigkeit des Filaments beziehen, so dass trotz Aufschmelzbarkeit der Hülle eine hohe Stabilität des Implantats gegeben ist.

Durch die vorbeschriebene Ausgestaltung wird eine Möglichkeit der Radialkraftsteigerung für geflochtene Stentimplantate möglich, ohne die Flexibilität einzuschränken. Diese basiert darauf, dass das Implantat beziehungsweise der Stent derart hinsichtlich seiner spezifischen Materialauswahl definiert ist, dass durch ein schonendes Heizen des Implantats, insbesondere der Hülle des Implantats, Kreuzungspunkte beziehungsweise Knotenpunkte der Implantatstruktur miteinander verbunden werden können, nach dem das Implantat in den Körper eingebracht wurde. Genauer wird es möglich, die Kreuzungspunkte des geflochtenen Stents postoperativ und nicht invasiv durch kurzzeitige elektromagnetische Aufheizung stoffschlüssig zu verbinden. Diese Aufheizung wird durch den Füllstoff, beziehungsweise bevorzugt die magnetisch aufheizbaren Nanopartikel (MNP) vermittelt, durch welche der Stent lokal und hochkontrolliert auf ein bestimmtes Temperaturniveau erwärmt werden kann.

Entsprechend sind im Sinne der vorliegenden Erfindung unter Knotenpunkten Bereiche beziehungsweise Punkte zu verstehen, welche unterschiedliche Stränge in direkter Nachbarschaft, insbesondere in Kontakt zueinander, aufweisen, und sind unter Kreuzungspunkte derartige Bereiche beziehungsweise Punkte zu verstehen, bei denen die zuvor benachbart vorliegende Stränge beziehungsweise Knotenpunkte miteinander stoffschlüssig verbunden sind.

Hierzu wird in der Hülle wie vorstehend definiert eine niedrigschmelzende Materialkomponente verwendet, welche durch eine entsprechende Aufheizung lokal, und gegebenenfalls unter Anpressdruck eines Ballonkatheters, aufgeschmolzen wird und somit die Fasern beim Abgleiten unter radialer Belastung hindert. Durch den sich einstellenden Abstand der Schmelzpunkte der Materialien der Hülle und des Kerns wird es dabei möglich, die Struktur des Kerns intakt zu lassen, so dass die grundsätzliche Stabilität nicht gemindert wird und ferner die dreidimensionale Struktur des Implantats durch das Aufschmelzen der Hülle nicht verändert wird.

Diese Art der postoperativen Verbindung der Kreuzungspunkte ist vorteilhaft, da der Stent für die minimalinvasive Freisetzung in einem Katheter komprimiert vorliegen muss und dort bereits verbundene Kreuzungspunkte die Flexibilität des Stents beschränken würden. Somit kann durch die Ausgestaltung des Implantats eine postoperative Stabilitätssteigerung insbesondere hinsichtlich einer Radialkraft hervorgerufen werden, wobei dennoch zum Einbringen des Implantats eine ausreichende Flexibilität vorliegt.

Die Erfindung löst somit das Problem der unzureichenden Radialkraft von Polymerstents. Auf diese Weise wird deren Anwendungsspektrum maßgeblich vergrößert. Metallische Stents, welche aktuell bevorzugt verwendet werden und Defizite insbesondere durch den dauerhaften Verbleib im Körper aufweisen, könnten dadurch zumindest teilweise ersetzt werden. Dieser Vorteil geht dabei nicht zu Lasten der Komprimierbarkeit des Stents, da eben die Stentstreben unter Last beim Einbringen noch einander abgleiten können und weiterhin die Möglichkeit eines minimalinvasiven Eingriffs ausbilden können.

Weitere Vorteile des Vorsehens des Füllstoffs in dem Mantel können somit darin gesehen werden, dass die Orientierbarkeit der Kernkomponente nicht durch den Füllstoff beziehungsweise die Nanopartikel gestört wird und ferner, dass über bildgebende Verfahren, wie beispielswiese mittels Magnetresonanztomographie (MRT) und/oder Magnetpartikel Bildgebung (Magnetic Particle Imaging, MPI) der Erfolg der Kreuzungspunktverbindung überprüfbar ist. Dies kann insbesondere deshalb von Vorteil sein, da wie vorstehend beschrieben eine Verbindung der Kreuzungspunkte postoperativ stattfindet und so grundsätzlich nicht auf einfache Weise ermittelbar ist, ob die Verbindung der Kreuzungspunkte erfolgreich war.

Um keine Schädigung von gesundem, umliegenden Gewebe hervorzurufen, kann die Schmelzpunkt des zweiten Matrixmaterials, also der zweite Schmelzpunkt, auf einen Temperaturbereich begrenzt sein, der eine Schädigung des umliegenden Gewebes verhindert.

Grundsätzlich kann der zweite Schmelzpunkt in einem Bereich liegen von ≥ 45 °C insbesondere bis ≤ 100 °C, beispielsweise ≥ 45 °C bis ≤ 75 °C. Dies stellt sicher, dass auch bei erhöhter Körpertemperatur die Struktur des Implantats intakt bleibt, jedoch zumindest bei kurzzeitiger Temperaturexposition keine Gewebeschädigung zu befürchten ist.

In einer Ausgestaltung kann der zweite Schmelzpunkt in einem Bereich liegen von ≥ 45 °C bis ≤ 65 °C. Insbesondere in dieser Ausgestaltung kann ein sehr schonendes Verbinden der Kreuzungspunkte erlaubt werden, da der Temperatureinfluss auf das umliegende Gewebe sehr gering ist und Temperaturen bis 65°C bei hier anwendbaren Expositionsdauern noch keine Gewebeschädigung hervorrufen beziehungsweise letzteres sicher verhindert werden kann.

Insbesondere, wenn der zweite Schmelzpunkt, also der Schmelzpunkt des zweiten Matrixmaterials in einem Bereich liegt von oberhalb von 50°C, beispielsweise in einem Bereich von > 65°C bis ≤ 100°C, kann zusätzlich zu dem Verbinden von Knotenpunkten, was bei einer Temperatur oberhalb des vorbenannten Schmelzpunktes realisierbar ist zusätzlich ein thermoablatives Verfahren durchgeführt werden. Hierzu können die Partikel beziehungsweise das Matrixmaterial des Mantels auf eine Temperatur beispielsweise in einem Bereich von 41°C bis 44°C erhitzt werden, ohne dass die Stabilität des Implantats gefährdet würde. Dabei kann es besonders vorteilhaft sein, dass erfindungsgemäß auch sehr enge Temperaturbereiche, wie etwa in einem Bereich von 41°C bis 44°C, oder auch andere insbesondere in den vorbenannten Bereichen liegende Temperaturbereiche sehr definiert einstellbar sind. Dies ist insbesondere möglich durch die Auswahl und Beladung der Füllstoffe und die Parameter deren magnetischer Anregung.

Unter den vorstehenden Temperaturbereichen könnte das Durchführen von thermoablativen Verfahren erlaubt werden, analog zu hochintensivem fokussierten Ultraschall (HIFU), hochfrequenzinduzierter Thermotherapie (HITT) oder laserinduzierter interstitieller Thermotherapie (LITT). Thermoablative Verfahren zielen auf eine Abtötung (Koagulation) des Zielgewebes, etwa von Tumorgewebe, mit Temperaturen von 41°C bis 44°C mit insbesondere einer apoptotischen Zellschädigung ab, wobei unerwünschte Nekrosen vermieden werden können. Das beschriebene Implantat kann bei thermoablativen Verfahren, insbesondere im tieferen Temperaturbereich, grundsätzlich zur Erzeugung von insbesondere therapeutisch effektiver Wärme oder zur Bildgebung dienen.

Hinsichtlich des ersten Matrixmaterials, also des Matrixmaterials des Kerns kann es bevorzugt sein, dass dieses ausgewählt ist aus der Gruppe bestehend aus Polylactiden (PLA), Polylactid-co-Glycolid (PLGA), Polyclycolide (PGA), Poly-4-Hydroxybutyraten (P4HB), Polydioxanon. Alternativ oder zusätzlich kann es vorgesehen sein, dass das zweite Matrixmaterial, also das Matrixmaterial der Hülle, ausgebildet ist aus der Gruppe bestehend aus Polycaprolacton (PCL), und einem Copolymer aus Polyglycolsäure und ε-Caprolacton, etwa aus 75 Gew.-% Polyglycolsäure und 25 Gew.-% ε-Caprolacton (PGCL). Es hat sich gezeigt, dass insbesondere diese Materialien sich bei der vorliegenden Erfindung vorteilhaft verwenden lassen, da sie entsprechende Stabilitäten ausbilden und ferner in den vordefinierten Temperaturbereichen der Schmelzpunkte liegen. Darüber hinaus sind diese Materialien biodegradierbar, so dass die Ausbildung biodegradierbarer Stents möglich ist. Schließlich lassen sich diese Materialien bevorzugt durch Coextrusion verarbeiten, was für das beschriebene Implantat ein bevorzugtes Herstellungsverfahren ist.

Es kann weiterhin bevorzugt sein, dass in dem zweiten Matrixmaterial einen Wirkstoff vorliegt. In dieser Ausgestaltung kann die Anwendungsbreite des Implantats weiter vergrößert werden. Grundsätzlich ist der Wirkstoff, der auf an sich bekannte Weise beispielsweise retardiert freigesetzt werden kann, frei wählbar und die Menge des Wirkstoffs in dem Mantel kann grundsätzlich abhängig sein von der gewünschten Anwendung.

Bevorzugt kann der magnetisch aufheizbare Füllstoff einen ferromagnetischen oder ferrimagnetischen, insbesondere superparamagnetischen, Füllstoff umfassen. Der superparamagnetische Effekt etwa von Nanoferriten beschreibt eine magnetische Eigenschaft sehr kleiner Partikel eines ferromagnetischen oder ferrimagnetischen Materials. Wenn diese auch bei Temperaturen unterhalb der Curie-Temperatur keine bleibende Magnetisierung aufweisen, nachdem ein zuvor angelegtes Magnetfeld abgeschaltet wurde, wird dies als superparamagnetischer Effekt bezeichnet. Eine Ansammlung von Nanoferriten in der Polymermatrix verhält sich daher makroskopisch wie ein Paramagnet, besitzt aber dennoch die hohe magnetische Sättigung eines Ferromagneten und reagiert dementsprechend wie ein weichmagnetischer Ferromagnet auf induktive Felder. Im Gegensatz zu einem Paramagneten sind es nicht einzelne Atome, sondern kleine magnetische Partikel, die ihre Magnetisierungsrichtung unabhängig voneinander verändern. Derartige superparamagnetische Stoffe, insbesondere superparamagnetische Nanoferritpartikel mit einer einstellbaren Sättigungstemperatur, werden somit bevorzugt genutzt, um die lokale Erwärmung zu steuern.

Vorteilhaft bei derartigen Füllstoffen kann es insbesondere sein, dass diese ein entsprechendes Aufheizen der Struktur mit einer einstellbaren Sättigungstemperatur besonders definiert und/oder in einem schnellen Zeitraum ermöglichen. Dadurch kann ein Erhitzen des Matrixmaterials und so ein Ausbilden von Verknüpfungspunkten mittels Aufschmelzen des Matrixmaterials unter Verwendung eines erfindungsgemäßen Implantats besonders schonend durchgeführt werden.

Beispiele für derartige superparamagnetische Füllstoffe umfassen insbesondere Ferrite, wie beispielsweise superparamagnetische Eisenoxid-Partikel, etwa Magnetit oder Maghemit.

Insbesondere kann es vorteilhaft sein, wenn der Füllstoff, insbesondere der superparamagnetische Füllstoff, eine Kristallitgröße, die auch als Kerngröße oder magnetische Kerngröße bezeichnet werden kann, aufweist in einem Bereich von größer oder gleich 3 nm bis kleiner oder gleich 100 nm, etwa größer oder gleich 10 nm bis kleiner oder gleich 30 nm, wobei die Kristallitgröße, bei welcher ein Material superparamagnetische Eigenschaften aufweist, stark materialabhängig sein kann. Derartige Nanopartikel, auch als magnetische Nanopartikel (MNP) bezeichnet, können durch ihre physikalischen Eigenschaften bei diagnostischen (Kontrastmittel in der Magnetresonanztomographie (MRT)) und therapeutischen Anwendungen aber auch bei einem Aufschmelzen des Mantels die beschriebenen Vorteile besonders effektiv erlauben.

Denn Nanopartikel neigen auf Grund ihrer magnetischen Anziehung sowie des großen Oberflächen-Volumen-Verhältnisses zur Bildung von Agglomeraten mit einer Größe von wenigen Mikrometern (makroskopische Agglomerate), beispielsweise von ≤ 10 µm. Bei der Herstellung von Nanocompositen kann die Bildung von Agglomeraten im Produktionsprozess nur bedingt bzw. mit hohem Aufwand beeinflusst werden. Die Agglomerate wirken wie Störstellen und beeinflussen maßgeblich die Eigenschaften der resultierenden Nanokomposite. Eine Möglichkeit zur Verbesserung der Werkstoffeigenschaften liegt in einer homogenen Verteilung der Partikel im Endprodukt. Zur Herstellung von Nanokompositen werden zurzeit zwei Herstellungsverfahren, der Schmelz-Mischprozess, sowie der Lösungs-Mischprozess, industriell angewendet. Zur Homogenisierung der Nanopartikel in der Matrix werden in-situ Polymerisation, Partikelfunktionalisierung oder Ultraschall-Wellen eingesetzt.

Vorteilhaft ist insbesondere der Spinnprozess, bei dem zur Herstellung des erfindungsgemäßen Implantats neben der partikelbeladenen funktionellen Komponente, des Mantels, eine zweite Komponente, der Kern, mit ausgesponnen wird, durch welche sowohl während des Spinnprozesses als auch nach Fertigstellung eine deutliche Verbesserung der mechanischen Festigkeit gewährleistet werden kann. Somit kommt insbesondere die Coextrusion zweier Materialien im Schmelzspinnprozess zum Einsatz.

Der Spinnprozess schließt sich an den Schmelzmischprozess, beispielsweise mit dem Doppelschneckenextruder, an. Dies kann einschrittig, aber auch zweischrittig ablaufen. Das Produkt des Schmelzmischprozesses, auch Compoundieren genannt, ist Granulat. Dieses wird dann anschließend im Schmelzspinnprozess zu Fasern ausgesponnen.

Es kann weiterhin bevorzugt sein, dass das Implantat eine tubuläre Struktur, also insbesondere eine rohrförmige beziehungsweise schlauchförmige Struktur, aufweist. Beispielsweise kann das Implantat ein Stent sein. Dabei kann somit das aus einer Kern-Mantel-Struktur aufgebaute Filament durch Faserverarbeitungsprozesse zu der tubulären Struktur verarbeitet werden.

Weiter vorteilhaft kann das Filament eine verkreuzte oder eine verschlungene Struktur aufweisen. Somit kann das Filament insbesondere durch an sich bekannte Faserverarbeitungsprozesse zum Implantat bearbeitet werden und dabei insbesondere kein Vlies sein. Dadurch lässt sich eine definierte und gleichermaßen stabile Struktur erzeugen, welche das Verwenden als Implantat verbessern kann.

Es kann insbesondere bevorzugt sein, dass das Filament eine geflochtene Struktur aufweist. Insbesondere eine geflochtene Struktur kann für eine Therapie in einem Hohlorgan oder in einem Gefäß Vorteile aufweisen. Denn so kann insbesondere ein textiler Stent in einer Geflechtstruktur ermöglicht werden, welche eine hohe Flexibilität aufweist und bei der Herstellung eine geringe mechanische Belastung der Fasern widerfährt. Darüber hinaus lässt sich insbesondere ein Geflecht problemlos komprimieren, um in ein Hohlorgan oder ein Gefäß eingeführt zu werden, und kann dabei in dem Hohlorgan aufgrund einer ausreichenden mechanischen Rückstellkraft seine gewünschte nicht-komprimierte Form erhalten. Dadurch kann ein Geflecht Vorteile gegenüber anderen Produkten aus faserverarbeitenden Verfahren oder auch gegenüber Vliesen beziehungsweise Vliesstoffen aufweisen. Ferner kann die Radialkraft wie vorstehend beschrieben durch die Verknüpfungspunkte weiter verbessert werden.

Es kann ferner bevorzugt sein, wenn in dem Mantel der magnetisch heizbare Füllstoff in einem Anteil vorliegt von größer oder gleich 0,1 Gew.-% bis kleiner oder gleich 90 Gew.-%, bevorzugt größer oder gleich 3 Gew.-% bis kleiner oder gleich 30 Gew.-%. Insbesondere in dieser Ausgestaltung kann der Füllstoff derart erwärmt werden, dass das Implantat auf die vorstehend beschrieben gewünschte Temperatur erhitzt wird. Dabei kann das Implantat gemäß der Erfindung insbesondere in dieser Ausgestaltung bei einer Monokomponentenstruktur, also ohne die Verstärkungsschicht, reduzierte mechanische Eigenschaften, wie etwa eine reduzierte Stabilität, aufweisen, so dass die vorliegende Erfindung insbesondere in dieser Ausgestaltung effektive Vorteile aufweisen kann.

Hinsichtlich weiterer technischer Merkmale und Vorteile des Implantats wird auf die Beschreibung der Anordnung, auf die Figuren und die Beschreibung der Figuren verwiesen, und umgekehrt.

Beschrieben wird ferner eine Anordnung aus einer Strahlungsquelle zum Emittieren von elektromagnetischer Strahlung und einem Implantat, wobei das Implantat einen magnetisch heizbaren Füllstoff aufweist. Die Anordnung ist dadurch gekennzeichnet, dass das Implantat wie zuvor beschrieben ausgestaltet ist.

Eine derartige Anordnung erlaubt es durch die Strahlungsquelle, dass sich der magnetisch aufheizbare Füllstoff in definierter und reproduzierbarer Weise durch magnetische Relaxationsprozesse induktiv aufheizt und das Implantat so definiert Verknüpfungspunkte durch Verschmelzen und einen entstehenden Stoffschluss unterschiedlicher Punkte des Implantats ermöglichen kann.

Dazu kann es insbesondere bei einer Verwendung im Körper von Vorteil sein, dass das Implantat und die Strahlungsquelle derart aufeinander abgestimmt sind, dass sich das Implantat durch von der Strahlungsquelle emittierte elektromagnetische Strahlung zumindest in dem Mantel auf eine Temperatur heizbar ist, die in einem Bereich liegt von ≥ 45 °C bis ≤ 100 °C. Dadurch kann beispielsweise eine effektive und gleichermaßen schonende Ausbildung von Verknüpfungspunkten beziehungsweise Kreuzungspunkten und gegebenenfalls Zerstörung von Krebsgewebe ermöglicht werden.

Ein entsprechendes Abstimmen von emittierter Strahlung auf das Implantat kann auf Seiten der elektromagnetischen Strahlung insbesondere durch das Einstellen einer geeigneten Frequenz ermöglicht werden. Beispielhafte Frequenzen und Feldamplituden umfassen einen Bereich von 10 kHz bis 1 MHz und 1 kA/m bis 100 kA/m.

Derartige Bereiche können vorteilhaft sein, da bei einer geeigneten Kombination von Frequenz und Feldamplitude, die unabsichtliche Erwärmung von Gewebe durch die Bildung von sogenannten Wirbelströmen besonders effektiv entgegengewirkt werden kann. Dabei wird insbesondere berücksichtigt, dass die Energiedeposition des Gewebes frequenzabhängig ist.

Ein entsprechendes Abstimmen von emittierter Strahlung, d. h. der Frequenz und Feldamplitude sowie der Richtung des Magnetfeldes, auf das Implantat kann auf Seiten des Implantats insbesondere erfolgen durch die Gestaltung der Teilcheneigenschaften, z. B. ihrer Größe, ihrer Kristallinität, ihres magnetischen Verhaltens, insbesondere ihrer magnetischen Relaxation, ihrem stabilisierenden Mantel, die sich auf die homogene Verteilung von kleinen bzw. keinen Agglomeraten im Polymer auswirkt. Die Gestaltung der Teilchen sieht auch eine Anordnung einzelner Teilchen im Polymer in Form einer Kette oder als Agglomerat vor, die die verstärkte Reaktion auf das angelegte Magnetfeld zur Folge hat.

Hinsichtlich weiterer technischer Merkmale und Vorteile der Anordnung wird auf die Beschreibung des Implantats, auf die Figuren und die Beschreibung der Figuren verwiesen, und umgekehrt.

Nachfolgend wird die Erfindung unter Bezugnahme auf die anliegenden Zeichnungen exemplarisch erläutert, wobei die nachfolgend dargestellten Merkmale sowohl jeweils einzeln als auch in Kombination einen Aspekt der Erfindung darstellen können, und wobei die Erfindung nicht auf die folgende Zeichnung, die folgende Beschreibung und das folgende Ausführungsbeispiel beschränkt ist.

Es zeigen:
Fig. 1 eine schematische Ansicht eines Filaments für ein Implantat gemäß der vorliegenden Erfindung; und
Fig. 2 die Wirkungsweise eines erfindungsgemäßen Implantats.

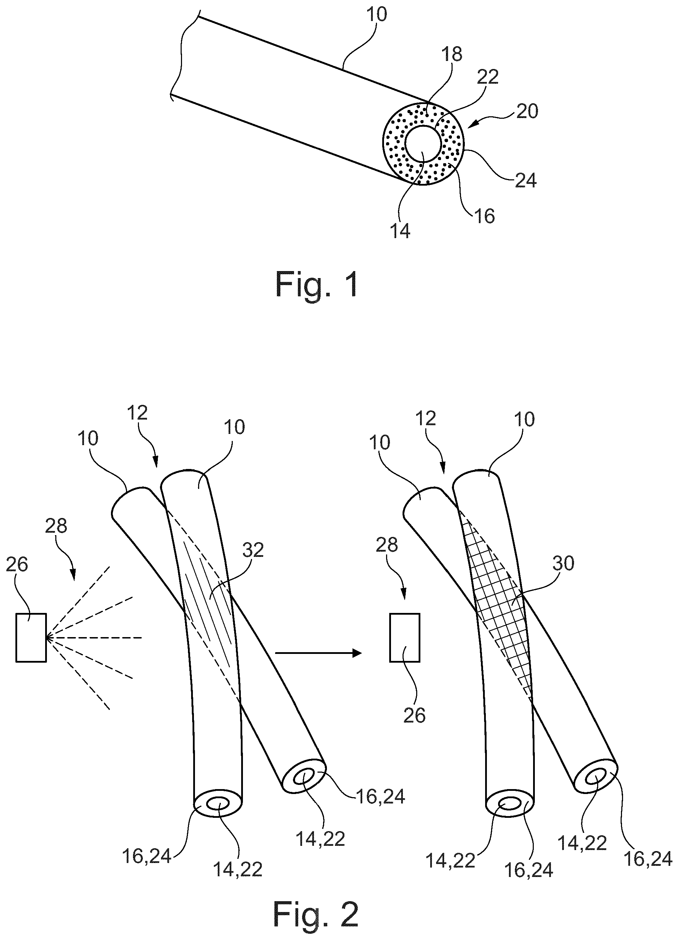

Figur 1 zeigt eine schematische Ansicht eines Filaments 10 für ein Implantat 12 gemäß der vorliegenden Erfindung. Das Implantat 12 dient insbesondere dem Implantieren in einen Körper, insbesondere in ein Hohlorgan eines Körpers. Darüber hinaus kann das Implantat 12 auch in ein Gefäß eingebracht werden eines Körpers eingebracht werden.

Das Implantat 12 ist aus dem Filament 10 aufgebaut, etwa in einer geflochtenen Struktur. Das Filament 10 weist wenigstens ein Matrixmaterial 14, 16 auf, in dem ein magnetisch heizbarer, insbesondere superparamagnetischer, Füllstoff 18 angeordnet ist und wobei wenigstens das zweite Matrixmaterial 16 ein Polymer umfasst. Ferner zeigt Figur 1, dass das Filament 10 einen Querschnitt mit einer Kern-Mantel-Struktur 20 aufweist.

Es ist gezeigt, dass der Kern 22 fadenförmig und eben als Faser beziehungsweise Filament ausgestaltet ist und der Mantel 24 eine schlauchförmige Struktur aufweist und den Kern 22 zumindest teilweise umhüllt.

Bei dem hier beschriebenen Implantat 12 ist es im Detail vorgesehen, dass der Kern 22 ein erstes insbesondere polymeres Matrixmaterial 14 mit einem ersten Schmelzpunkt aufweist und dass der Mantel 24 ein zweites polymeres Matrixmaterial 16 mit einem zweiten Schmelzpunkt aufweist, wobei der magnetisch heizbare Füllstoff 18 zumindest in dem zweiten Matrixmaterial 16 vorliegt. Der zweite Schmelzpunkt ist niedriger, als der erste Schmelzpunkt, insbesondere wobei die Schmelzpunktdifferenz zwischen dem ersten Schmelzpunkt und dem zweiten Schmelzpunkt in einem Bereich liegt von ≥ 5 °C und wobei der zweite Schmelzpunkt in einem Bereich liegt von ≥ 45 °C insbesondere bis ≤ 100 °C. Hierdurch ergibt sich verbesserte Anwendbarkeit des Implantats 12.

Es ist ferner zu erkennen, dass die Beladung des Füllstoffs 18 in dem Mantel 24 größer ist, als im Kern 22. Insbesondere ist der Kern 22 frei von dem Füllstoff 18. Ferner ist die Kern-Mantel-Struktur 20 beziehungsweise das Filament 10 insbesondere als Zweischichtstruktur ausgebildet.

Für eine Herstellung des Implantats 12 wird der Füllstoff 18, wie beispielsweise werden die zu verwendenden Nanoferrite, synthetisiert und zusammen mit dem Polymer auf einem Doppelschneckenextruder zu einem spinnbaren Compound compoundiert. Anschließend wird dieser Compound mit dem Schmelzspinnprozess zu induktiv-aufheizbaren Fasern versponnen. Zum Erzeugen der Kern-Mantel-Struktur 20 wird insbesondere eine Coextrusion von Kernmaterial und Mantelmaterial ausgeführt. Das Implantat 12, wie insbesondere der Stent, wird über ein Kathetersystem bis an die entsprechende Stelle im Körper beziehungsweise im Hohlorgan oder Gefäß vorgeschoben und anschließend mittels Selbstexpansion oder Ballondilatation zur Ausübung eines gewissen Anpressdrucks expandiert. Hierbei wird von einer guten Flexibilität des Implantats 12 beim Einbringen profitiert.

Nach einem Einbringen des Implantats 12 in ein Hohlorgan oder ein Gefäß eines Körpers kann das Implantat 12 mittels eines Strahlungsquelle 26 zum Emittieren von elektromagnetischer Strahlung bearbeitet werden, wie nachfolgend beschrieben.

Bei Anregung in einem elektromagnetischen Feld wandelt der Füllstoff 18 die aufgenommene Energie des Feldes in Wärme um und geben diese an die Umgebung ab. Dies wird etwa möglich unter Verwendung eben der Strahlungsquelle 26, welche elektromagnetische Strahlung derart emittiert, dass sich der Füllstoff 18 auf eine geeignete Temperatur erhitzt, um das zweite Matrixmaterial 16 aufzuschmelzen. Die Strahlungsquelle 26 kann mit dem Implantat 12 eine zusammenhängende beziehungsweise aufeinander abgestimmte Anordnung 28 ausbilden.

Bei einem beschriebenen Implantat 12 kann die Stabilität postoperativ, also nach dem Einbringen in ein Hohlorgan eines Körpers, verbessert werden. Im Detail kann die Radialkraft über die Ausbildung einer stoffschlüssigen Verbindung und somit dem Ausbilden von Kreuzungspunkten 30 erreicht werden. Dies wird wie vorstehend angedeutet realisiert, indem sich der Mantel 24 über den Schmelzpunkt des zweiten Matrixmaterials 16 erhitzt und so zuvor lose Kontaktpunkte 32 durch ein Aufschmelzen und Erhärten des zweiten Matrixmaterials 16 Kreuzungspunkte 30 ausbilden. Es sei erwähnt, dass im Sinne der vorliegenden Erfindung die Begriffe Kreuzungspunkte 30 und Kontaktpunkte 32 breit zu verstehen sind und entsprechende Bereiche hiervon umfasst sein sollen.

Das Ausbilden der Kreuzungspunkte 30 kann zu einem Zeitpunkt realisiert werden, an dem das Implantat 12 nicht mehr komprimierbar sein muss. Es wird beispielsweise zunächst ohne verbundene Kreuzungspunkte 30 in einen Katheter geladen und minimalinvasiv an die zu behandelnde Position gebracht. Dort wird das Implantat 12 expandiert, beispielsweise mittels Selbstexpansion oder Ballondilatation, und erst nach der Expansion werden die Kontaktpunkte 32 des Filaments 10 unter Ausbildung von Kreuzungspunkten 30 stoffschlüssig verbunden, wie in Figur 2 gezeigt.

Erfindungsgemäß ergibt sich so eine besonders vorteilhafte Kombination einer problemlosen minimalinvasiven Einbringbarkeit des Implantats 12 durch eine hohe Flexibilität und einer anschließenden Radialkraftserhöhung unter Ausbildung von Kreuzungspunkten 30 im Hohlorgan.

### Bezugszeichen

- 10: Filament
- 12: Implantat
- 14: erstes Matrixmaterial
- 16: zweites Matrixmaterial
- 18: Füllstoff
- 20: Kern-Mantel-Struktur
- 22: Kern
- 24: Mantel
- 26: Strahlungsquelle
- 28: Anordnung
- 30: Kreuzungspunkte
- 32: Kontaktpunkt

## Patentansprüche

1. Implantat (12) zum Implantieren in einen Körper, insbesondere in ein Hohlorgan oder ein Gefäß eines Körpers, wobei das Implantat (12) aus einem Filament (10) aufgebaut ist, das wenigstens ein polymeres Matrixmaterial (14, 16) aufweist, in dem ein magnetisch heizbarer Füllstoff (18) angeordnet ist, wobei das Filament (10) einen Querschnitt mit einer Kern-Mantel-Struktur (20) aufweist, **dadurch gekennzeichnet, dass** der Kern (22) ein erstes, insbesondere polymeres, Matrixmaterial (14) mit einem ersten Schmelzpunkt aufweist und dass der Mantel (24) ein zweites, polymeres Matrixmaterial (16) mit einem zweiten Schmelzpunkt aufweist, wobei der magnetisch heizbare Füllstoff (18) zumindest in dem zweiten Matrixmaterial (16) vorliegt, wobei der zweite Schmelzpunkt niedriger ist, als der erste Schmelzpunkt, wobei der zweite Schmelzpunkt in einem Bereich liegt von ≥ 45 °C insbesondere bis ≤ 100 °C.

2. Implantat (12) nach Anspruch 1, **dadurch gekennzeichnet, dass** der zweite Schmelzpunkt in einem Bereich liegt von ≥ 45 °C bis ≤ 65 °C.

3. Implantat (12) nach einem der Ansprüche 1 oder 2, **dadurch gekennzeichnet, dass** der zweite Schmelzpunkt in einem Bereich liegt von > 65 °C bis ≤ 100 °C.

4. Implantat (12) nach einem der Ansprüche 1 bis 3, **dadurch gekennzeichnet, dass** eine Schmelzpunktdifferenz zwischen dem ersten Schmelzpunkt und dem zweiten Schmelzpunkt in einem Bereich liegt von ≥ 5 °C, beispielsweise ≥ 20 °C.

5. Implantat (12) nach einem der Ansprüche 1 bis 4, **dadurch gekennzeichnet, dass** das erste Matrixmaterial (14) und das zweite Matrixmaterial (16) biodegradierbar sind.

6. Implantat (12) nach einem der Ansprüche 1 bis 5, **dadurch gekennzeichnet, dass** das erste Matrixmaterial (14) ausgewählt ist aus der Gruppe bestehend aus Polylactiden, Polylactid-co-Glycoliden, Polyglycoliden, Poly-4-Hydroxybutyraten, Polydioxanon.

7. Implantat (12) nach einem der Ansprüche 1 bis 6, **dadurch gekennzeichnet, dass** das zweite Matrixmaterial (16) ausgewählt ist aus der Gruppe bestehend aus Polycaprolacton und einem Copolymer aus Polyglycolsäure und ε-Caprolacton.

8. Implantat (12) nach einem der Ansprüche 1 bis 7, **dadurch gekennzeichnet, dass** in dem zweiten Matrixmaterial (16) einen Wirkstoff vorliegt.

9. Implantat (12) nach einem der Ansprüche 1 bis 8, **dadurch gekennzeichnet, dass** der Kern (22) frei ist von dem Füllstoff (18).

10. Implantat (12) nach einem der Ansprüche 1 bis 9, **dadurch gekennzeichnet, dass** die Kern-Mantel-Struktur (20) durch einen Coextrusionsprozess erzeugt ist.

11. Implantat (12) nach einem der Ansprüche 1 bis 10, **dadurch gekennzeichnet, dass** der magnetisch aufheizbare Füllstoff (18) einen ferromagnetischer oder ferrimagnetischer, insbesondere superparamagnetischer, Füllstoff umfasst.

12. Implantat (12) nach einem der Ansprüche 1 bis 11, **dadurch gekennzeichnet, dass** das Filament (10) eine geflochtene Struktur aufweist.

13. Anordnung (28) aus einer Strahlungsquelle (26) zum Emittieren von elektromagnetischer Strahlung und einem Implantat (12), wobei das Implantat (12) einen magnetisch heizbaren Füllstoff (18) aufweist, **dadurch gekennzeichnet, dass** das Implantat (18) ausgestaltet ist nach einem der Ansprüche 1 bis 12.

14. Anordnung (28) nach Anspruch 13, **dadurch gekennzeichnet, dass** das Implantat (12) und die Strahlungsquelle (26) derart aufeinander abgestimmt sind, dass das Implantat (12) durch von der Strahlungsquelle (26) emittierte elektromagnetische Strahlung zumindest in dem Mantel (24) auf eine Temperatur heizbar ist, die in einem Bereich liegt von ≥ 45 °C insbesondere bis ≤ 100 °C.

15. Anordnung (28) nach einem der Ansprüche 13 oder 14, **dadurch gekennzeichnet, dass** die Strahlungsquelle (26) zum Emittieren von Strahlung einer Frequenz in einem Bereich von 10 kHz bis 1 MHz bei einem Feldamplitudenbereich von 1 kA/m bis 100 kA/m eingerichtet ist.

## Claims

1. Implant (12) for implanting in a body, in particular in a hollow organ or a vessel of a body, wherein the implant (12) is constructed from a filament (10) containing at least one polymer matrix material (14, 16) in which a magnetically heatable filler (18) is arranged, wherein the filament (10) has a cross section with a core-sheath structure (20), **characterized in that** the core (22) comprises a first, in particular polymer, matrix material (14) having a first melting point and **in that** the sheath (24) comprises a second, polymer matrix material (16) having a second melting point, wherein the magnetically heatable filler (18) is present at least in the second matrix material (16), wherein the second melting point is lower than the first melting point, wherein the second melting point is in a range from ≥ 45°C in particular to ≤ 100°C.

2. Implant (12) according to claim 1, **characterized in that** the second melting point is in a range from ≥ 45°C to ≤ 65°C.

3. Implant (12) according to claim 1 or 2, **characterized in that** the second melting point is in a range from > 65°C to ≤ 100°C.

4. Implant (12) according to any of claims 1 to 3, **characterized in that** a melting point difference between the first melting point and the second melting point is in a range of ≥ 5°C, for example ≥ 20°C.

5. Implant (12) according to any of claims 1 to 4, **characterized in that** the first matrix material (14) and the second matrix material (16) are biodegradable.

6. Implant (12) according to any of claims 1 to 5, **characterized in that** the first matrix material (14) is selected from the group consisting of polylactides, polylactide-co-glycolides, polyglycolides, poly-4-hydroxybutyrates, polydioxanone.

7. Implant (12) according to any of claims 1 to 6, **characterized in that** the second matrix material (16) is selected from the group consisting of polycaprolactone and a copolymer of polyglycolic acid and ε-caprolactone.

8. Implant (12) according to any of claims 1 to 7, **characterized in that** an active ingredient is present in the second matrix material (16).

9. Implant (12) according to any of claims 1 to 8, **characterized in that** the core (22) is free from the filler (18).

10. Implant (12) according to any of claims 1 to 9, **characterized in that** the core-sheath structure (20) is created by a coextrusion process.

11. Implant (12) according to any of claims 1 to 10, **characterized in that** the magnetically heatable filler (18) comprises a ferromagnetic or ferrimagnetic, in particular superparamagnetic, filler.

12. Implant (12) according to any of claims 1 to 11, **characterized in that** the filament (10) has a braided structure.

13. Arrangement (28) of a radiation source (26) for emitting electromagnetic radiation and an implant (12), wherein the implant (12) contains a magnetically heatable filler (18), **characterized in that** the implant (12) is configured according to any of claims 1 to 12.

14. Arrangement (28) according to claim 13, **characterized in that** the implant (12) and the radiation source (26) are matched to one another in such a way that the implant (12) is heatable, at least in the sheath (24), to a temperature in a range from ≥ 45°C in particular to ≤ 100°C by electromagnetic radiation emitted by the radiation source (26).

15. Arrangement (28) according to claim 13 or 14, **characterized in that** the radiation source (26) is set up to emit radiation at a frequency in a range from 10 kHz to 1 MHz in a field amplitude range of 1 kA/m to 100 kA/m.

## Revendications

1. Implant (12) destiné à être implanté dans un corps, en particulier dans un organe creux ou un vaisseau d'un corps, l'implant (12) étant composé d'un filament (10) qui présente au moins un matériau de matrice polymère (14, 16), dans lequel est disposée une matière de remplissage pouvant être chauffée magnétiquement (18), le filament (10) présentant une section transversale avec une structure noyau-enveloppe (20), **caractérisé en ce que** le noyau (22) présente un premier matériau de matrice, en particulier polymère, (14) ayant un premier point de fusion et **en ce que** l'enveloppe (24) présente un deuxième matériau de matrice polymère (16) ayant un deuxième point de fusion, la matière de remplissage pouvant être chauffée magnétiquement (18) étant présente au moins dans le deuxième matériau de matrice (16), le deuxième point de fusion étant inférieur au premier point de fusion, le deuxième point de fusion se situant dans une plage de ≥ 45 °C en particulier jusqu'à ≤ 100 °C.

2. Implant (12) selon la revendication 1, **caractérisé en ce que** le deuxième point de fusion se situe dans une plage de ≥ 45 °C à ≤ 65 °C.

3. Implant (12) selon l'une des revendications 1 ou 2, **caractérisé en ce que** le deuxième point de fusion se situe dans une plage de > 65 °C à ≤ 100 °C.

4. Implant (12) selon l'une des revendications 1 à 3, **caractérisé en ce qu'**une différence de point de fusion entre le premier point de fusion et le deuxième point de fusion se situe dans une plage de ≥ 5 °C, par exemple ≥ 20 °C.

5. Implant (12) selon l'une des revendications 1 à 4, **caractérisé en ce que** le premier matériau de matrice (14) et le deuxième matériau de matrice (16) sont biodégradables.

6. Implant (12) selon l'une des revendications 1 à 5, **caractérisé en ce que** le premier matériau de matrice (14) est choisi dans le groupe constitué par les polylactides, les polylactide-co-glycolides, les polyglycolides, les poly-4-hydroxybutyrates, la polydioxanone.

7. Implant (12) selon l'une des revendications 1 à 6, **caractérisé en ce que** le deuxième matériau de matrice (16) est choisi dans le groupe constitué par la polycaprolactone et un copolymère d'acide polyglycolique et d'ε-caprolactone.

8. Implant (12) selon l'une des revendications 1 à 7, **caractérisé en ce qu'**un principe actif est présent dans le deuxième matériau de matrice (16).

9. Implant (12) selon l'une des revendications 1 à 8, **caractérisé en ce que** le noyau (22) est exempt de matière de remplissage (18).

10. Implant (12) selon l'une des revendications 1 à 9, **caractérisé en ce que** la structure noyau-enveloppe (20) est fabriquée par un processus de coextrusion.

11. Implant (12) selon l'une des revendications 1 à 10, **caractérisé en ce que** la matière de remplissage (18) pouvant être chauffée magnétiquement comprend une matière de remplissage ferromagnétique ou ferrimagnétique, en particulier superparamagnétique.

12. Implant (12) selon l'une des revendications 1 à 11, **caractérisé en ce que** le filament (10) présente une structure tressée.

13. Agencement (28) composé d'une source de rayonnement (26) pour émettre un rayonnement électromagnétique et d'un implant (12), l'implant (12) présentant une matière de remplissage (18) pouvant être chauffée magnétiquement, **caractérisé en ce que** l'implant (18) est réalisé selon l'une des revendications 1 à 12.

14. Agencement (28) selon la revendication 13, **caractérisé en ce que** l'implant (12) et la source de rayonnement (26) sont accordés l'un à l'autre de telle sorte que l'implant (12) peut être chauffé par un rayonnement électromagnétique émis par la source de rayonnement (26), au moins dans l'enveloppe (24), à une température se situant dans une plage de ≥ 45 °C en particulier jusqu'à ≤ 100 °C.

15. Agencement (28) selon l'une des revendications 13 ou 14, **caractérisé en ce que** la source de rayonnement (26) est configurée pour émettre un rayonnement d'une fréquence dans une plage de 10 kHz à 1 MHz avec une plage d'amplitude de champ de 1 kA/m à 100 kA/m.
